# EUROPEAN PATENT SPECIFICATION

(11) **EP 2 566 551 B1**
(45) Date of publication and mention of the grant of the patent: **30.03.2016**
(21) Application number: 11729491.8
(22) Date of filing: 13.04.2011
(51) Int. Cl.: A61M 15/00

(54) **INHALATION DEVICE**
INHALIERGERÄT
INHALATEUR

(30) Priority: 20.04.2010 TR 201003091; 13.04.2010 TR 201002877
(43) Date of publication of application: 13.03.2013
(73) Proprietor: Sima Patent ve Lisanslama Hizmetleri Ltd.Sti., Esenler/Istanbul (TR)
(72) Inventor: BILGIC, Mahmut, Esenler-Istanbul (TR)
(86) International application number: PCT/TR2011/000094
(87) International publication number: WO 2011/129794

(56) References cited:
- US-A1- 2008 308 102
- US-A1- 2009 078 252
- US-A1- 2009 139 516
- US-A1- 2010 059 052

## Description

### Field of the Invention

The present invention relates to an inhaler used in delivering medicament in dry powder form to patients by the inhalation route. In addition, the present invention relates to an inhaler providing deliver dry powder medicament as stored in blister packages effectively and safely, and the method for using said device.

### Description of the Prior Art

It is well known to use inhalers for delivering medicaments utilized in the treatment and prophylaxis of respiratory diseases by the inhalation route. Inhalation treatment is the most commonly preferred treatment method in these diseases as the inhalers provide ease of use; the medicaments have a more rapid onset of time resulting from local administration and they have fewer side effects. Inhalers have been designed in order to provide effective and sufficient delivery of the medicaments used in the treatment of respiratory diseases, particularly in asthma and chronic obstructive pulmonary disease. These inhalers vary according to their operating mechanisms and the physical form of the medicament to be delivered.

In the inhalers used to deliver drugs in dry powder form, the drug is carried in reservoirs, capsules or blisters packages. It is important to deliver each dose to the patient with exact accuracy and preciseness since the required medicament dose in the inhalation is very low. Inhalation devices comprising blister packages, which are one of the inhaler types utilized for the inhalation of the medicaments in dry powder form mentioned above, are commonly used. The blister package is comprised of a plurality of blister pockets each of which contains medicament in dry powder form. In response to each actuation of the inhaler to realize the inhalation, the blister package is indexed from one part of the device to the other; one of the blister pockets is opened and one dose of the dry powder medicament becomes ready for inhalation subsequent to the delamination of the blister package or the perforation of its by piercing components in the device.

The dry powder medicament is provided to be entrained with the air entering the blister pocket which is opened upon the inhalation of the patient and reach the lungs passing through the air ways of the patient. Thanks to the blister packages they comprise, this type of inhaler can be used repeatedly at certain intervals without requiring any additional operations to place the blister pocket containing one dose of the medicament in dry powder form before the inhalation and the moisture effects on the dry powder medicament in the blisters are prevented in this way. In addition, blister packages enable the dry powder medicament to last for long periods of time by protecting it from gasses like oxygen, which affect the transmission characteristics of the device as well as environmental factors such as UV rays.

It is highly significant to deliver a sufficient amount of the dry powder medicament contained in the blister pocket which is opened to realize an effective inhalation in the inhalers comprising blister packages to the lungs in each actuation. In addition to the dry powder medicament's physical and chemical characteristics such as suitable aerodynamic particle size, suitable particle shape, uniformity of the particle size distribution, low aerodynamic dispersion forces, low density, high physical and chemical stability, the mechanism and the specifications of the inhaler are of the factors affecting the efficiency of the inhalation. The strong effect of the medicaments, which are used in inhalation treatment, at low doses highlights the significance of controlled dosing in each inhalation during the inhalation treatment.

Under dosing of the dry powder medicament administered to the patient during inhalation operation results in the absence of desired effects while overdosing results in undesirable side effects. Thus, it is required that the necessary amount of the dry powder medicament is delivered to the patient during inhalation for the desired effect to be observed in the treatment.

A wide range of inhalers have been designed to enable inhalation of the medicament in dry powder form from blisters.

The inhalation device marketed under the trade mark Diskus® by GlaxoSmithKlein is one of the most well-known inhalers on the market. In Diskus® inhaler, each blister pocket of the blister package is prepared for inhalation with the slide mechanism. Before each inhalation, the mouthpiece cover is slid and the mouthpiece and the slide are exposed. In this device, the lever attached to the slide triggers the gear mechanism while the slide is being rotated from one end to the other; the blister package is indexed in the device; the blister pocket is opened and the medicament in dry powder form becomes ready for inhalation.

Some of the devices present in the state of the art are summarized below;

US 2008/308102 discloses a manifold that is suitable for use in a medicament dispenser for dispensing dry powder medicament. The dry powder medicament can be in a blister pack. The manifold assists effective release of medicament powder for inhalation by a patient, for example from an opened blister pocket to a mouthpiece of the dispenser, and then it can be inhaled by a patient. The device comprises a manifold which directs inhaled air from chimney exit to the chamber inlet via the open blister pocket thereby entraining the dry powder.

US 2009/078252 discloses a fault sensing system for dry powder inhalers. The document mainly aims to detect breakage or some other sort of failure (such as stretching or delamination or tearing) of the blister strip to prevent the user from using the device although it no longer delivers the dry powder.

US 2010/059052 discloses a device for inhalation of dry powder medicament from blister pockets on a blister strip. The document aims to provide a mechanism that would notify the user when the blister sheet used inside the device has damaged. For this purpose the inventors devised a sheet driver that includes an indicator pin that "pops out" when loss of pulling force is experienced. The indicator pis has been designed in a way that when the blister sheet is damaged the pin pops out and engages with one or more of the elements of the device hence preventing the movement of the gear mechanism. This, as a result, gives a clear indication of a fault to the patient.

When current inhalers are taken into consideration, there is need for inhalers having a mechanism which provides the patient to make sure that the required amount of the dry powder medicament for the treatment is inhaled during each inhalation.

According to this, the inventor has designed a safety inhaler which comprises a blister package and enables controlled dose intake of the medicament in dry powder form during each inhalation.

The present invention relates to the inhaler which comprises blister package and has a mechanism that guarantees the blister package to be accurately and precisely positioned in response to each actuation of the device.

### Summary of the Invention

An inhaler suitable for delivery of the medicament in dry powder form according to the present invention comprising;
a blister package composed of a plurality of blister pockets which are spaced at equal intervals and each of which comprises medicament in dry powder form;
a mouthpiece enabling the patient to inhale the medicament in dry powder form from the opened blister;
a gear mechanism enabling the blister package to be indexed and the medicament in dry powder form to become ready for inhalation;
a rotatable mouthpiece cover hiding the mouthpiece and triggering the gear mechanism;
a drive gear which is a component of the gear mechanism and is joined with the mouthpiece cover via side covers, and
a housing situated between the upper housing member and the lower housing member in which the blister package and the gear mechanism are enclosed characterized by:
   side covers which provide a connection between each end of the drive gear and the appropriate connection point of the mouthpiece cover so as to enable the drive gear and the mouthpiece cover to synchronize, and at least one stopper that hinders the rotation of at least one gear in the gear mechanism.

Each of the side covers that passes through one connection point of the mouthpiece cover engages with one end of the drive gear and this enables the mouthpiece cover to be connected to the drive gear by two ends of said drive gear in a tight and reliable way. Therefore, in each actuation of the inhaler, the mouthpiece cover and the drive gear rotate together and synchronously and the rotational movement of the mouthpiece cover is accurately transmitted to the other components of the gear mechanism by the drive gear. In addition, due to the connection between each end of the drive gear and an appropriate connection point of the mouthpiece cover, the mouthpiece cover is connected with the gear mechanism by the drive gear in a simple and cost-effective way.

Said side covers allows the mouthpiece cover and the drive gear to be on the same component, and therefore to synchronize. Thus, the constant-angle rotational movement of the mouthpiece cover is accurately transmitted to the other components of the gear mechanism by the drive gear.

The inhaler of the present invention is an easy-grip, manual device which is suitable to be used for the delivery of the medicament in dry powder form.

The housing of the device of the present invention has been designed such that each component of the blister package and the gear mechanism, which have a significant role in enabling the device to work properly, is situated accurately and works harmoniously. To this end, the housing is divided into several compartments. The used portion and the unused portion of the blister package are accommodated in separated compartments in order to prevent the medicament in dry powder form remaining in the opened blister pocket spilling the other components of the housing. Furthermore, the housing also comprise a beak which enables the blister package to be peeled and a manifold through which the dry powder medicament in the open blister passes before reaching the mouthpiece during inhalation. The housing can be in any appropriate shape, while it is preferably elliptic or circular.

The upper and the lower housing members interlock with each other and enclose the housing in order to keep the housing and the gear mechanism fixed together. The mouthpiece cover hiding the mouthpiece is rotated by being slid on the upper and lower housing members. The grids on the surface of the lower and the upper housing members provide effective actuation by preventing the slipping of the finger while rotating the mouthpiece cover. The upper and the lower housing members can be in any appropriate shape which provides ease of use.

The mouthpiece cover hiding the mouthpiece of the device of the present invention has been designed such that it also actuates the device. When the upper and the lower housing members of the device joined together, engagement tabs on the inside surface of the lower housing member engage with engagement recesses on the inside surface of the upper housing member and the upper and lower housing members are fixed tightly. In addition, the protrusions on the upper and the lower housing members are joined end to end and form a restricted path where the mouthpiece cover rotates. Before each inhalation, both the mouthpiece is uncovered and one dose of the medicament in dry powder form becomes ready for inhalation as one of the blister pockets is opened as a result of the mouthpiece cover being manually rotated along the path restricted by joining the protrusions on the upper and the lower housing members. The rotational path on which the cover moves is restricted on both ends by the protrusions of the upper and the lower housing members. The constant-distance path that the protrusions of the upper and the lower housing members define results in the mouthpiece cover being rotated by a fixed angle in the range of 30° to 160°, preferably, 50° to 120°, most preferably 55°,60°,65°, 70°,75°,80° to 92.5°,95°,97.5°, 100°,102.5°,105°,107.5°,110°,112.5°, 115° in response to each actuation of the device.

The mouthpiece cover that triggers the gear mechanism of the device can be found in one of two positions. The mouthpiece cover can easily be switched from the first position to the second position with the help of a carved part in one end. When the mouthpiece cover is in the first position, the mouthpiece cover resides on the protrusion in one end of the rotational path. When the first position is on, the mouthpiece cover is completely covered and the device is in standby mode. When the mouthpiece cover is in the second position, the mouthpiece cover resides on the protrusion in the other end of the rotational path and one dose of the dry powder medicament becomes ready for inhalation upon the actuation of the device.

In each actuation of the inhalation device, if the blister package is not indexed or positioned properly then the blister pocket is not opened completely and as a result less than the required amount of the dry powder medicament is inhaled; or if more than one blister pocket is opened more than the required amount of the dry powder medicament is inhaled, which may result in dangerous complications for the patient. Therefore, it is highly significant to provide the blister to be positioned at accurate position and to be completely opened in each actuation of the inhalation device.

According to the present invention, the mouthpiece cover of the device is joined with a gear mechanism by the drive gear via two connection points. For each end of the drive gear to make a fixed and tight connection with the connection point of the mouthpiece cover, one side cover is used for each end of the drive gear. The ends of the drive gear are carved so the ends of the side covers can tightly engage with them from inside and hence the ends of the drive gear can tightly interlock with the ends of the side covers.

The shape of the inside face of the carved part on both ends of the drive gear matches with the shape of the end of the side cover that fits into this carved part. Each connection point of the mouthpiece cover is a hole having a shape matching with the shape of the end of the side cover that passes through this connection point. Therefore, each of the side covers passes through one connection point of the mouthpiece cover as it engages with one end of the drive gear and this enables the mouthpiece cover to be connected to the drive gear by two ends of said drive gear in a tight and reliable way and to synchronize with the drive gear. Therefore, the rotational movement of the mouthpiece cover is accurately transmitted to the gear mechanism by the drive gear.

In each actuation of the inhalation device, the gear mechanism is triggered by the rotation movement of said mouthpiece cover from the first position to the second position along the rotational path restricted on both ends by the protruding parts on the upper and the lower housing members to provide the blister package to be indexed. In each actuation of the device, the constant-distance path that the protrusions of the upper and the lower housing members define allows said mouthpiece cover to be rotated by a fixed angle. Accordingly, because of the side covers that enable the mouthpiece cover to be connected to drive gear by two ends of said drive gear, in each actuation of the inhalation device, only rotation of said mouthpiece cover from the first position to the second position on the constant-distance path guarantees that the blister package is indexed by the same distance and the opened blister is at accurate position so that the sufficient amount of the dry powder formulation contained in the opened blister can be inhaled. In addition to this, the patient makes sure whether the blister is opened completely by controlling the position of the mouthpiece cover on the constant-distance path. In other words, if the mouthpiece cover is in the second position wherein it resides on the protruding part on the other end of the constant-distance rotational path, the patient makes sure that one blister is completely opened and one dose of the dry powder medicament contained in the opened blister becomes ready for inhalation; if the mouthpiece cover is between the first position and the second position, the patient makes sure that one blister is not completely opened.

One end of the drive gear passes through the center of the lower housing member and is tightly joined with the mouthpiece cover by a side cover in one connection point while the other end passes through the center of the upper housing member and is joined with the mouthpiece cover by the other side cover in the other connection point.

On each connection point of the mouthpiece cover, there is a stabilizing resilient cover. Extensions under the stabilizing resilient covers pass through holes on the upper or the lower housing members, according to the position of the connection point and ensure the stabilizing resilient covers that they connect with remain stable. The rotation of the mouthpiece cover is prevented from both sides as a pawl under each of the stabilizing resilient covers in both sides of the device interlock with the mouthpiece cover. Before inhalation, the resilient parts of each stabilizing resilient cover that match with the shape of the fingers are pressed to raise the pawls and release the mouthpiece cover in order to move the mouthpiece cover which actuates the device. Thus, the mouthpiece cover can easily be rotated when the resilient parts of each stabilizing resilient cover on both sides of the device that match with the shape of the fingers are pressed on. When the resilient parts of the stabilizing resilient covers are not pressed, the pawls under the stabilizing resilient covers do not allow the mouthpiece cover to move.

According to the present invention, each gear of the gear mechanism in the device directly or indirectly engages with each other. The drive gear, which is one of the components of the gear mechanism, provides the mouthpiece cover to trigger the gear mechanism.. In each actuation of the device, the constant-angle rotational movement of the cover is transmitted to gear of a indexing ratchet wheel which interlocks with the indexing wheel via the drive gear. The indexing wheel synchronizes with the indexing ratchet wheel when the mouthpiece cover is switched from the first position to the second position. The indexing wheel is engaged with the winding wheel gear and the pinion gear and the rotation of the indexing wheel gear causes them to move as well. The mechanism wheel engages with the inside of the winding wheel gear via the arms of the mechanism wheel. The counter gear that engages with the small gear under the base gear rotates by means of the base gear that engages with the pinion gear and the counter gear with movement of the mouthpiece cover. Therefore, with the rotation of the indexing wheel gear, both the lid sheet of the blister package is provided to be coiled on the wings of the winding wheel as the winding wheel is rotated by the mechanism wheel that engages with the winding wheel gear, and the counter gear that engages with the small gear under the base gear is provided to be rotated by means of the base gear that engages with the pinion gear. After the counter gear is rotated, the new number of unused blisters can be seen through the display aperture. According to the invention, the mouthpiece cover triggers the gear mechanism by the drive gear. As a result of the engagement of the both ends of the drive gear with the connection points via the side covers, the rotational movement of the mouthpiece cover is exactly transmitted to the gear mechanism. This connection between the mouthpiece cover and the drive gear is strong. The side covers synchronize provide the mouthpiece cover and the drive gear to synchronize the same component, and therefore the rotational movement of the mouthpiece cover is exactly transmitted to the other components of the gear mechanism by the drive gear. Accurate transmission of the rotation of the mouthpiece cover to the gear mechanism enables the gear mechanism to work properly and the blister package to be positioned accurately.

According to the present invention, the indexing wheel may be another component of the gear mechanism. The recesses of the indexing wheel match with the shape of the blister package. The fact that the blister pockets of the blister package are received in these recesses in sequence while the indexing wheel rotates allows the blister package to be indexed properly. The rotation angle of the indexing wheel depends on the number of the recesses of the indexing wheel. In each actuation of the inhalation device, the indexing wheel can be rotated by 15° to 120°.According to the invention, there are preferably 8 recesses of the indexing wheel. Therefore, the indexing wheel is supposed to rotate by 45° in response to each actuation of the device for the opened blister pocket to be positioned accurately. The drive gear which engages with the mouthpiece cover via the side covers accurately transmits the constant rotational movement, by an angle in the range of 30°to 160° of the mouthpiece cover to the gear of the indexing ratchet wheel when the device is actuated. As the arms of the indexing ratchet wheel interlock with the indexing wheel from the inside, the indexing wheel and the indexing ratchet wheel synchronize. Therefore, the constant-angle rotation that the mouthpiece cover executes when the device is actuated causes the indexing wheel to rotate by a constant angle of 45°.The length of the path that the mouthpiece cover follows and the constant angle value is adjusted such that the indexing wheel rotates by45°in response to each actuation of the device. Thus ,the blister package is completely opened for the inhalation of the sufficient amounts of the medicament in dry powder form as a result of the accurate positioning of the blister package in response to each actuation of the device of the present invention.

At least one component which is situated in the lower housing member and serves as a stopper engages with the teeth of at least one of the gears of the gear mechanism and stabilizes the gear in a suitable position in order to prevent backward rotation of the blister package. While the stopper can hinder the rotation of any gear of the gear mechanism, it preferably hinders the rotation of the indexing ratchet wheel interlocked in the indexing wheel. The stopper can be anywhere suitable in the lower housing member and have any shape. Consequently, the connection between the mouthpiece cover and the drive gear via the side covers and the stopper interlocking with the teeth of the indexing ratchet wheel provide the blister package to be accurately and precisely positioned in response to each actuation of the device. Thus, controlled dosing of the dry powder medicament in the opened blister pocket is enabled when the device is actuated.

The term "controlled dosing" refers to the intake of the medicament in dry powder form on the required amount.

The counter gear in the device pertaining to the present invention displays the number of the unused blister pockets remaining in the device. In response to the actuation of the device by the mouthpiece cover, the mouthpiece is uncovered, the blister package is indexed and one dose of the dry powder medicament is prepared for inhalation while the counter gear rotates as well.

On the counter gear, there exist numerals equal to the number of the blister pockets present in the device and they are spaced at equal angles. In a device comprising 60 doses, the angle between the numerals is approximately 5°. The counter gear rotates as a result of the reflection of the rotation of the indexing wheel gear via the pinion gear and the base gear. In response to the each actuation of the device, rotation of the indexing wheel by the same angle each time due to the accurate transmission of the movement of the mouthpiece cover to the gear mechanism via the drive gear results in the rotation of the counter gear approximately by the same angle as well and each numeral on the counter gear is clearly seen through the display aperture on the upper housing member. Therefore, the patient is sure of the number of the unused blister pockets remaining in the device.

Before the inhalation, the device is actuated by the mouthpiece cover being rotated along the rotational path, and the drive gear which is joined with the both connection points of the mouthpiece cover, via the side covers, precisely transmits the movement of the mouthpiece cover to the gear of the indexing ratchet wheel because of the side covers. The indexing ratchet wheel interlocks with the indexing wheel from inside via its arms and enables the indexing wheel to rotate 45° in each actuation of the device. Upon the indexing wheel's movement, the blister package is indexed and is peeled by means of the beak present in the housing. As the indexing wheel gear engages with the winding wheel gear and the pinion gear, these gears synchronize with the indexing wheel. Since the mechanism wheel engages both with the winding wheel gear and the winding wheel, the rotation of the winding wheel gear causes the winding wheel to move and the lid sheet of the blister package coils tightly on the winding wheel. The ppinion gear engages with the base gear while the small gear under the base gear engages with the counter gear. In brief, the movement of the mouthpiece cover along the rotational path causes the blister package to be accurately positioned as a result of the rotation of the indexing wheel by 45° and the number of the unused blisters remaining in the device is clearly seen through the display aperture as the counter gear rotates by 5°.

The inhaler according to the present invention comprises a blister package composed of a plurality of blister pockets each of which comprises medicament in dry powder form and which are spaced at equal intervals. The blister package carries the medicament in dry powder form in one-dose portions and it is preferably a blister strip and it is preferably peelable. The blister pockets comprised in the blister package are spaced in equal intervals and each of them carries one dose of the medicament in dry powder form.

While the blister package is indexed on the indexing wheel, the beak on the housing peels the blister. Therefore, one dose dry powder medicament becomes ready for inhalation after the blister package is peeled open in each actuation of the device.

The base sheet of the blister package on which the blister cavities are spaced is accumulated in the separated compartment of the housing. The lid sheet that provides impermeability of the blister package, on the other hand, is coiled on the winding wheel, which is one of the components of the gear mechanism positioned in the other side of the housing. The winding wheel has a plurality of resilient wings. The resilient wings of the winding wheel has been designed such that they balance the force of attraction on the lid sheet of the blister package applied by the winding wheel by eliminating the augmentation emerging in the diameter of the winding wheel as a result of the lid sheet of the blister package coiling on the wings of the winding wheel. The rotation of the winding wheel is induced by the rotation of the winding wheel gear, which the winding wheel engages with, by the indexing wheel when the device is actuated. The mechanism wheel that engages with the winding wheel gear causes the winding wheel to rotate unidirectionally.

The blister pocket that is opened as a result of the beak's peeling the blister package is situated immediately under the manifold. Upon the inhalation by the patient, the airflow that enters the device through at least one air inlet on the upper housing member entrains the dry powder medicament in the opened blister pocket via the manifold to the mouthpiece and enables the delivery of said medicament to the patient. The air inlet on the upper housing member that allows the entry of air can be in any suitable shape and size that enables external air to enter the device easily and at convenient speed.

The air inlet that the external air flow passes through is preferably designed not to be close where the patient holds the device in order not to prevent air flow. Furthermore, in order to deliver the required amount of the dry powder medicament in the opened blister to the patient, the air inlet is designed such that it allows the entry of the airflow through the air inlet at a convenient angle.

One end of the manifold communicates with the opened blister while the other end communicates with the inlet. On the one end of the manifold that communicates with the blister, at least two apertures with four sub-apertures are situated. Upon the inhalation of the patient, some of the air flow which enters through the air inlet passes through one of these apertures and entrains the medicament in dry powder form through the other aperture to the manifold. There is preferably a tapered channel between the manifold and the mouthpiece to connect these components with each other. The medicament in dry powder form that is entrained to the manifold with the airflow is delivered to the patient via the mouthpiece. The manifold can be in any appropriate shape though it is preferably longer than 1mm.

The mouthpiece is designed to fit the mouth for the patient to comfortably inhale the medicament in dry powder form. According to the shape of the device, the mouthpiece can be in any suitable shape or size as well as being fixed or movable. Furthermore, it could be attached or unattached to the upper and/or the lower cover.

Each component of the device pertaining to the present invention can be made of any suitable material though they are preferably be made of plastic. These plastics can preferably be chosen from a group comprising the types of styrene acrylonitrile, polyoxymethylene acetale, acrylic-polymethylmetacrylate, cellulose acetate, polyetheretherketone, polyvinyl choloride, polyethylene, polypropylene, acrylonitrile butadiene styrene, polycarbonate, polyamide, polystyrene, polyurathane or fluoropolymer while it is preferably polyoxymethylene acetale. The plastic components can be manufactured by methods like injection molding. Moreover, each component can be in any suitable color.

According to the present invention, the lid and base sheets composing the blister package are sealed very tightly by at least one of the methods comprising cold formed bonding, hot metal bonding, hot metal welding, radio frequency welding, laser welding or ultrasonic welding in order to provide impermeability, more preferably by cold formed bonding method. Since these cold formed bonding methods can be carried out at lower temperatures than hot sealing methods, they are the most appropriate methods to use in the case that the medicament carried in the blister is heat sensitive. According to the present invention, the lid and the base sheets constituting the blister package preferably consist of a plurality of layers. Polymeric layers, aluminum foil and preferably fluoropoylmer film are among the layers that form the lid and the base sheet.

Fluoropolymer film is a polymeric film which is used in blister packs and provides excellent moisture barrier. This chemically inert polymeric film does not cause any change in the taste of the formulation when it is in contact with the dry powder formulation. In addition, it easily constitutes a layered structure with the other polymeric layers which are composed of various polymers. It is appropriate to be transacted with heat.

For preserving the stability of the dry powder formulation stored in the blister package, preferably at least one of the polymeric layers comprises at least one desiccant agent including silica gel, zeolite, alumina, bauxite, anhydrous calcium sulfate, activated carbon and clay which has the property of water absorption in order to decrease gas and moisture permeability of the layer.

In order to provide high moisture and gas protection, aluminum foil can be used in lid and base sheets of blister packs. These aluminum foils must be thick enough to provide the desired protection for the stability of the moisture sensitive dry powder formulation stored in the blister cavity while they can be chosen to be preferably in the range of 5 to 80 µm, more preferably in the range of 15 to 65 µm.

The polymeric layers in the lid and the base sheets of the blister pack mentioned in the present invention are made of the same or different polymers. The thickness of these polymeric layers varies according to the type of the polymeric substance used and its properties while they are preferably in the range of 5-100 µm, more preferably in the range of 15-60 µm.

In the blister package, the layer which is in contact with the dry powder medicament in the blister cavity is preferably a polymeric layer. Due to the porous structure of aluminum foil and electrostatic forces, some of the dry powder formulation sticks onto the inside layer of the blister cavity, and hence it may cause uncontrolled dosing.

The term "uncontrolled dosing" refers to the intake of the medicament in dry powder form less or more than the required amount.

The polymers composing the polymeric layer are preferably selected from thermoplastics such as polyethylene, polypropylene, polystyrene, polyolefin, polyamide, polyvinyl chloride, polyurethane or synthetic polymers.

The blister pockets in the blister package can be in any appropriate shape. The plurality of blister pockets spaced at equal intervals on the base sheet of the blister package can be the same or different shape, structure or volume.

The reference numbers of the drawings added to exemplify the present invention and the detailed description of the invention according to these drawings are given below but the scope of the invention should not be limited to these drawings.

### In the Drawings

Figure 1 is a perspective view of an inhaler according to the present invention;
Figure 2 is an exploded view of the inhaler of the invention;
Figure 3 is a perspective view of the blister pack for use with the inhaler of the invention;
Figures 4a and 4b are perspective views of the housing of the inhaler according to the invention;
Figures 5a and 5b are perspective views of upper and lower housing members of the inhaler according to the invention;
Figure 6a is a perspective view of the mouthpiece cover of the inhaler of the invention;
Figure 6b is an exploded view of the communication between the mouthpiece cover, the drive gear and the stabilizing resilient covers in the inhaler of invention;
Figure 6c is a cross-sectional view of the communication between the mouthpiece cover, the drive gear and the stabilizing resilient covers in the inhaler of the invention;
Figure 6d is a cross-sectional view of the communication between the mouthpiece cover, the drive gear and the stabilizing resilient covers in the inhaler of the invention;
Figure 6e is an exploded view of the communication between the drive gear and the side covers in the inhaler of the invention;
Figure 6f is a cross-sectional view of the connection of the stabilizing resilient cover with the lower housing member in the inhaler of the invention;
Figures 7a-7c are cross-sectional views of the engagement of the gears composing the gear mechanism with each other in the inhaler of the present invention;
Figure 8 is a cross-sectional view of the blister package delaminating in course of the operation of the inhaler of the present invention;
Figure 9 is a perspective view of the counter gear used in the inhaler of the present invention.

### Detailed Description of the Drawings

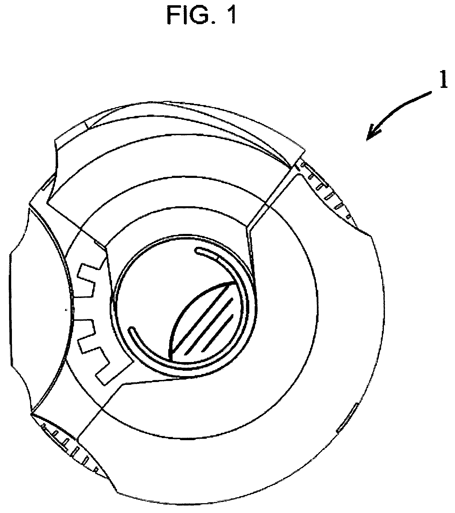

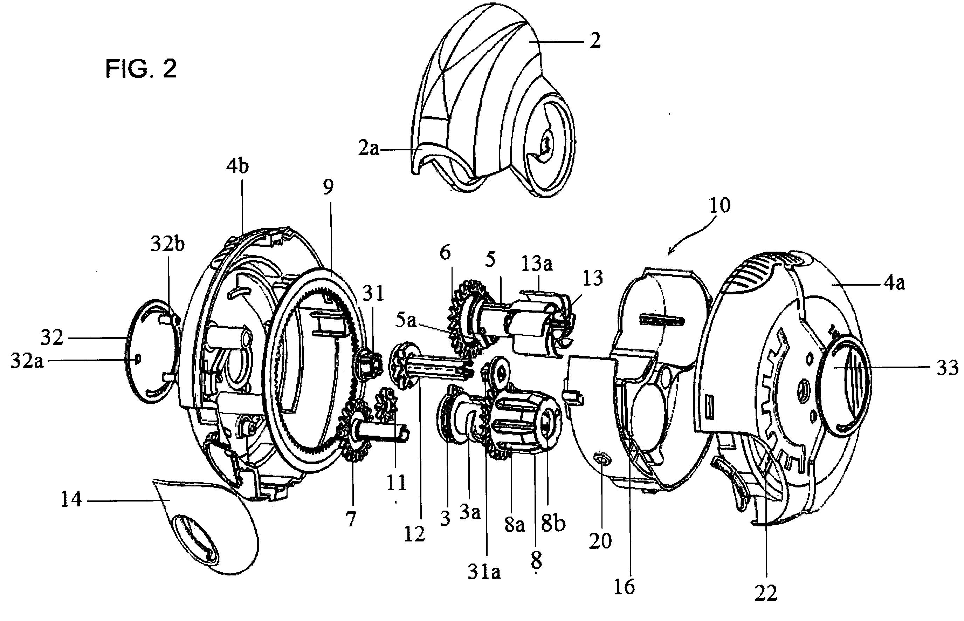

The inhaler (1) of the present invention comprises a gear mechanism situated in the housing (10) between an upper housing member (4a) and a lower housing member (4b) in order to enable the inhalation of the dry powder medicament carried in a blister package (15), as displayed in figures 1a, 1b and 2. Each component of the inhaler (1) is positioned at particular spots in the housing (10) to guarantee proper and accurate working.

The inhaler (1) of the present invention shown in figure 1 is ready for inhalation. In this case, a mouthpiece cover (2) is in a second position and the cover of the mouthpiece (2) is completely exposed. The mouthpiece cover (2) is rotated by holding the carved part (2a) on one end of the mouthpiece cover (2) in order to switch to a first position, wherein the mouthpiece is completely covered, from the second position. In this way, the mouthpiece (14) is completely exposed while the mouthpiece cover (2) is switched from the first position to the second position and the gear mechanism is triggered by a drive gear (12). The drive gear (12) precisely transmits the movement of the mouthpiece cover (2) to an indexing ratchet wheel (3).

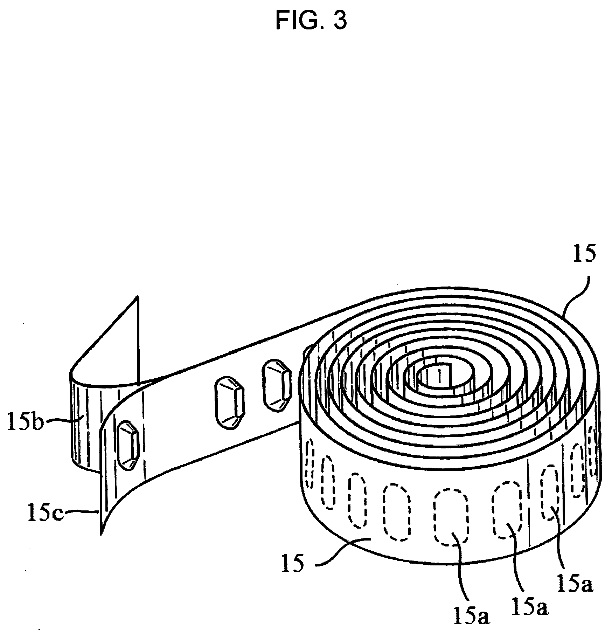

An indexing wheel (8) engages with the indexing ratchet wheel (3) and enables a blister package (15) shown in figure 3 to be indexed. Blister pockets (15a) composing the blister package are received in recesses (8a) on the indexing wheel and the blister package (15) is indexed when the indexing wheel (8) rotates. In the inhaler (1) of the present invention, the shapes of these recesses (8a) on the indexing wheel (8) have been designed to match with the shapes of the blister pockets (15) composing the blister package (15) for the blister package to be indexed properly.

The blister package (15) shown in figure 3 is composed of a lid sheet (15b) which provides impermeability and a base sheet (15c) on which the blister pockets (15a) are spaced at equal intervals. Each blister pocket contains medicament in dry powder form comprising one or more active agents.

The rotational movement that the mouthpiece cover (2) of the device for actuation of the inhaler executes while switching from the first position to the second is transmitted to the indexing ratchet wheel (3) via the drive gear (12) that the mouthpiece cover (2) engages with. As displayed in figure 2, arms (3a) of the indexing ratchet wheel interlock with the protrusions (8b) inside the indexing wheel (8) and rotate the indexing wheel (8) unidirectionally. Therefore, the blister package (15) is indexed forward while the indexing wheel (8a) rotates as the blister pockets (15a) composing the blister package (15) are received in the recesses (8a) of the indexing wheel. A beak (16) in the housing (10) peels the blister package (15) while the blister package (15) is indexed and provides one blister pocket (15a) to be opened in response to each actuation of the device (1). The beak (16) peels the blister package (15) with the help of a sharp edge (16a) that is in contact with the lid sheet of the blister package (15b).

The winding wheel gear (6), which is another component of the gear mechanism, engages with the gear of the indexing wheel (8) as displayed in figure 2. The mechanism wheel (5) that interlocks the winding wheel (13) from inside has arms (5a) to interlock with the interior teeth of the winding wheel gear (6). Therefore, the mechanism wheel (5) engages both with the winding wheel (13) and the winding wheel gear (6) and moves with these component in actuation of the inhaler (1). When the indexing wheel (8) rotates the winding wheel gear (6), the winding wheel (13) rotates unidirectionally owing to the arms (5a) of the mechanism wheel interlocking with the interior teeth of the winding wheel gear (6) and the lid sheet (15b), which is peeled away by the beak (16) while the blister package is indexed, is tightly coiled on the wings (13a) of the winding wheel. The base sheet (15c) of the blister package (15) where the blister pockets are spaced is accumulated in a separate part (18a) of the device.

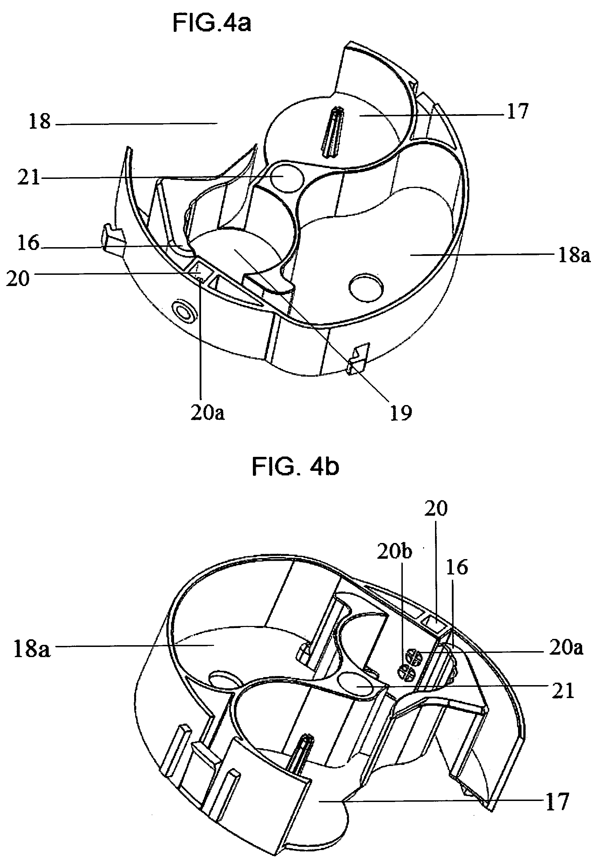

Different perspective views of the housing (10) wherein the gear mechanism and the other components of the inhaler (1) of the present invention are arranged are displayed in figures 4a and 4b. Furthermore, as can be seen in figures 4a and 4b, the housing (10) also comprises components having significant roles in the actuation of the device such as the beak (16), the manifold (20), and apertures with four sub-apertures (20a, 20b). Each component comprised in the housing is situated in an appropriate part of the housing (10) in order to enable the inhaler (1) to work properly. The drive gear (12) passes through the center (21) of the housing and engages with the mouthpiece cover (2) at two points. The blister package (15) is in the lower part (17) of the housing and coiled up. In response to each actuation of the device (1), the blister package (15) is peeled by the beak (16) in the housing while being indexed by the indexing wheel (8) situated in the upper part (19) of the housing. The lid sheet (15b) of the blister package (15), which provides impermeability is indexed over the beak (16) and coiled on the wings of the winding wheel (13a) which is situated in the side part (18) of the housing. The base sheet (15c) of the blister package (15) on which the blister pockets (15a) are spaced, on the other hand, is accumulated in the separated compartment (18a) of the housing (10). Upon the inhalation by the patient, air passes through the aperture with four sub-apertures (20a), under the manifold (20) and into the opened blister pocket. The air entrains the dry powder medicament contained in the opened blister pocket (15a) in response to each actuation of the device; and passes through the other aperture with four-sub-apertures (20b) and reach the mouthpiece via the manifold (20).

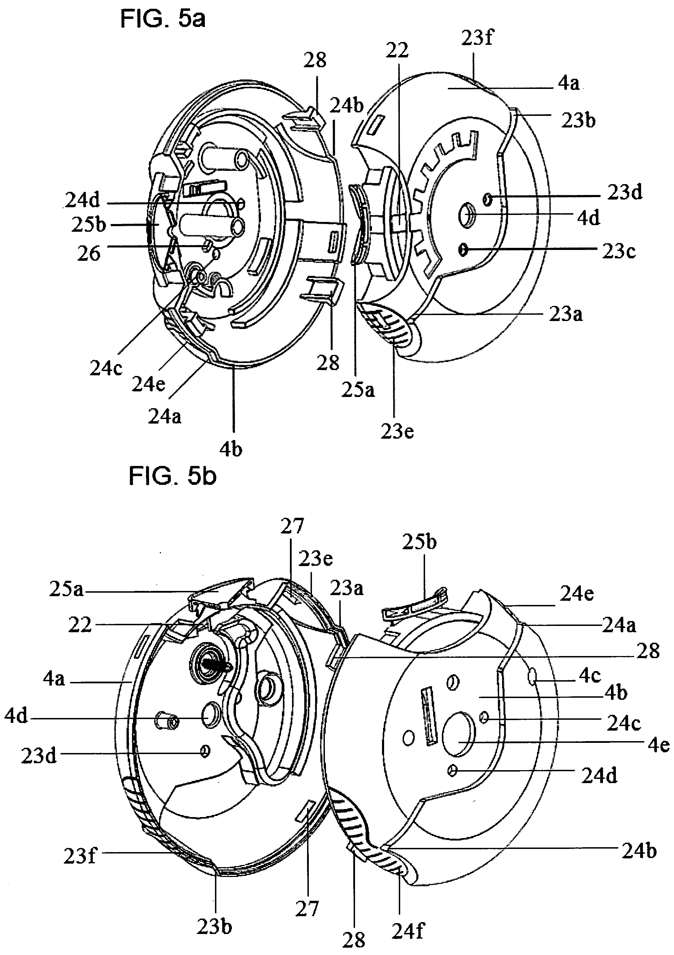

The housing (10) and the other components of the inhaler (1) pertaining to the present invention are kept together as upper housing member (4a) and lower housing member (4b) displayed in figures 5a and 5b are joined together. Engagement tabs (28) on the inside surface of the lower housing member (4b) engage with engagement recesses (27) on the inside surface of the upper housing member (4a) and the upper and lower housing members are fixed tightly. Therefore, the protrusions (23a; 23b) on the upper housing member (4a) and the protrusions (24a, 24b) on the lower housing member (4b) are joined end to end and define a restricted path for the rotational movement of the mouthpiece cover (2). In each actuation of the device, the constant-distance path that the protrusions (23a, 23b, 24a, 24b) of the upper and the lower housing members (4a, 4b) define allows said mouthpiece cover (2) to be rotated by a fixed angle. The mouthpiece cover (2) can be moved from the first position to the second position along this restricted path. When the mouthpiece cover (2) is in a first position, the mouthpiece (14) is completely covered, the device is in standby mode and the mouthpiece cover (2) leans on a first protrusion (23a) on the upper housing member and the first protrusion (24a) on the lower housing member. The mouthpiece (14) is manually slid along the rotational path with the help of the carved part (2a) to switch to a second position. The mouthpiece (14) is completely exposed when the cover (2) is in this position, one dose of the dry powder medicament is ready for inhalation and the mouthpiece cover (2) leans on a second protrusion (23b) on the upper housing member and a second protrusion (24b) on the lower housing member.

As displayed in figures 5a and 5b, one half (25a) of the tapered channel that interconnects the manifold (20) with the mouthpiece (14) is comprised in the upper housing member (4a) while the other half of it (25b) is comprised in the lower housing member (4b). The channel is constituted as a whole when the upper (4a) and the lower (4b) housing members are joined together. Upon the inhalation by the patient, air that enters the device through at least one air inlet (22) arranged in the upper housing member (4a) passes through the aperture with four sub-apertures (20a), reaches the opened blister (15a) and entrains the dry powder medicament there to the manifold (20) by passing it through the other aperture with four sub-apertures (20b). Grids on the upper housing member (23e, 23f) grids on the lower housing member (24e, 24f) prevent a slip of the finger while rotating the mouthpiece cover.

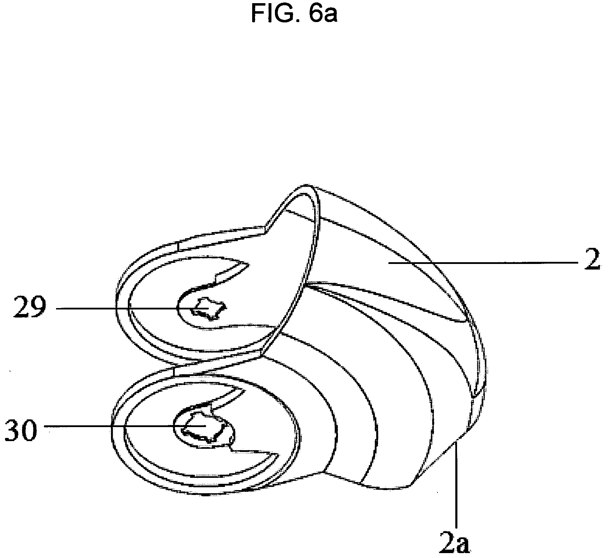

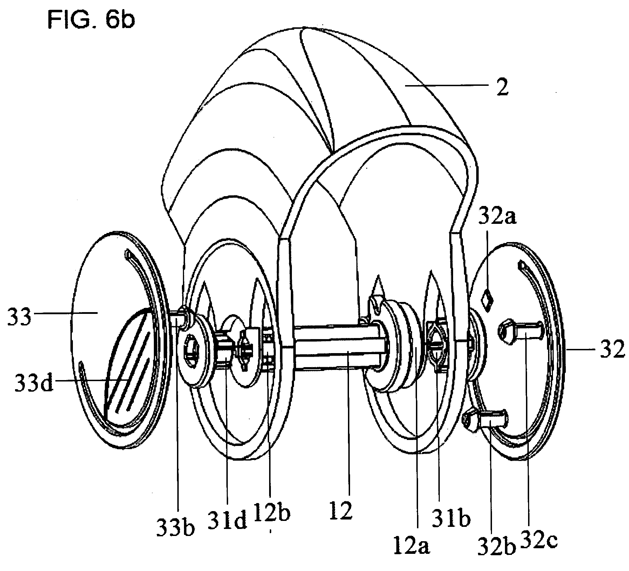

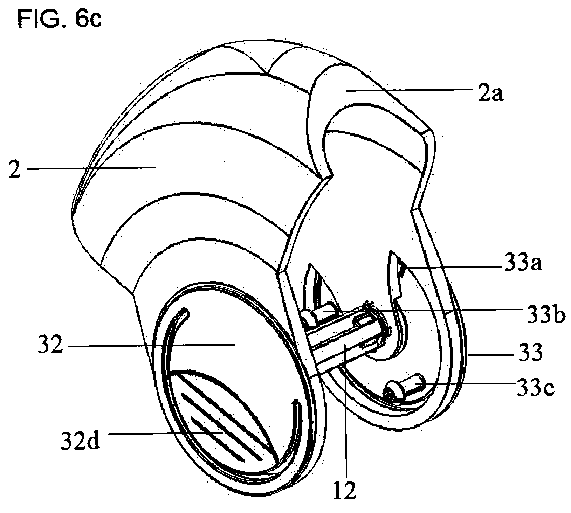

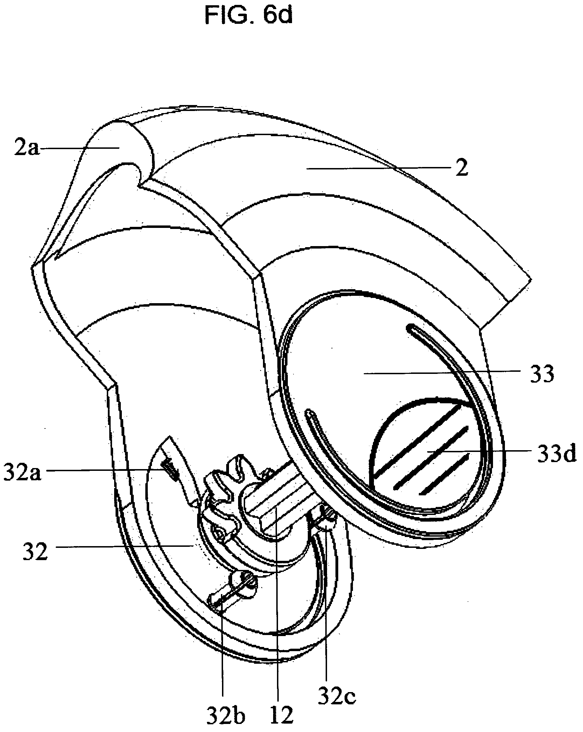

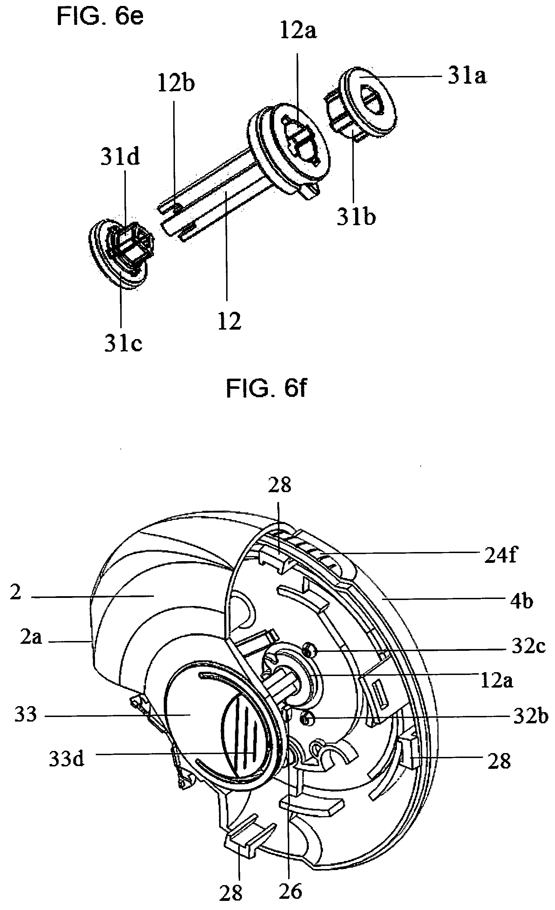

The mouthpiece cover (2) of the inhaler pertaining to the present invention is displayed in figure 6a. A carved part (2a) in one end of the device easy manual movement of the mouthpiece cover. The mouthpiece cover (2) is joined with the gear mechanism via connection points. The drive gear (12) is joined with the connection points (29, 30) of the mouthpiece cover via the side covers (31a, 31c) as can be seen in figures 6b, 6c and 6d, illustrating the connection between the mouthpiece cover (2), the drive gear (12), side covers (31a, 31c) and stabilizing resilient covers (32,33). Each of these side covers (31a; 31c) passes through the center (4d) of the upper housing member or the center (4e) of the lower housing member and joins with the end (12a; 12b) of the drive gear. It can be seen in figure 6d that the both ends (12a; 12b) of the drive gear are carved such that the end of the side cover (31b; 31d) can pass through. Each end of the side covers (31d; 31b) passes through one of the connection points (29; 30) of the mouthpiece cover and it is received in the recess in one end (12b; 12a) of the drive gear, thus it tightly and stably interconnects the mouthpiece cover (2) with the drive gear (12). The mouthpiece cover (2) synchronizes with the drive gear (12) as the connection point (29; 30) of the mouthpiece cover which matches with the ends (31d; 31b) of the side covers that passes through it on both sides of the device and the end (12b; 12d) of the drive gear that it communicates with are on the same component.

As is seen from figures 6a-6e, the shapes of the ends (31b; 31d) of the side covers that are received in the carved parts on the ends of the drive gear and the shapes of the connection points (29, 30) of the mouthpiece cover are not identical since the two ends (12a, 12b) of the drive gear are not identical.

The mouthpiece cover (2) rotates by the same angle each time on the constant-distance path that is composed by splicing the protrusions (23a, 23b) on the upper housing member and the protrusions (24a, 24b) on the lower housing member illustrated in figures 5a and 5b while switching from the first position to the second position. The angle of the rotational movement of the mouthpiece cover varies depending on the shape and the size of the device but it is a constant value in the range of 30° to 160°. Since the both ends of the drive gear (12a; 12b) interconnect with the respective connection point (30; 29) of the mouthpiece cover via the side cover (31a: 31c), the mouthpiece cover (2) and the drive gear (12) synchronize and the rotational movement of the mouthpiece cover (2) by a constant angle in the range of 30° to 160°, in response to each actuation of the device causes the drive gear (12) to rotate by a constant angle each time. Thus, the rotation of the drive gear (12) by a constant angle causes the indexing wheel (8) to rotate by the same angle via the indexing ratchet wheel (3) in response to each actuation of the device.

On the indexing wheel (8) illustrated in figure 2, there are preferably 8 recesses (8a) and the angles between these recesses are equal. Therefore, the indexing wheel (8) rotates by 45° each time the device is actuated for the blister pockets that are received in these recesses (8a) to be positioned accurately. The blister package (15) indexed by the 45° rotation of the indexing wheel (8) in response to each actuation of the device is peeled by the beak (16) and one dose of the dry powder medicament becomes ready for inhalation when one blister pocket is opened.

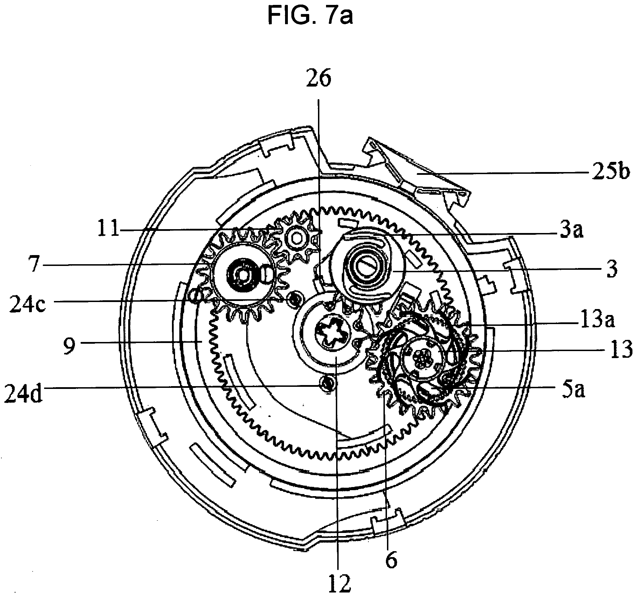

There is one stopper (26) in the lower housing member in order to provide the opened blister in the blister package (15) which is indexed by the indexing wheel (8) to be positioned precisely (figure 5). Figure 7a shows that the stopper (26) interlocks with the tooth of the indexing ratchet wheel (3) and hinders its rotation. The rotational movement of the mouthpiece cover (2) by the same angle each time the device (1) is actuated is precisely transmitted to the gear of the indexing ratchet wheel (3) by the drive gear (12) that joins with the connection points (29; 30) of the mouthpiece cover via the side covers (31c; 31b), and therefore the indexing wheel (8) which engages with the indexing ratchet wheel (3) is rotated by the same angle each time the device (1) is actuated. The stopper component (26) positioned in the lower housing member (4b) prevents backward movement of the blister package (15) which is indexed by the indexing wheel (8) that synchronizes with the indexing ratchet wheel (3) by keeping the position of the indexing ratchet wheel (3) stable.

There is one stabilizing resilient cover (33; 32) on each connection point (29; 30) of the mouthpiece and each side cover, as displayed in figures 2, 6a-6d, and 6f. When the mouthpiece cover (2) is in the first position, pawls (32a, 33a) under the stabilizing resilient covers, which are on the connection points (29, 30) of the mouthpiece, interlock with the mouthpiece cover (2) on both sides, as seen in figures 6c and 6d. The pawl (33a) under the stabilizing resilient cover that is on the first connection point (29) interlocks with the mouthpiece cover on one side (figure 6c). The pawl (32a) under the stabilizing resilient cover that is on the second connection point (30) of the mouthpiece cover interlocks with the mouthpiece cover (2) on the other side (figure 6d). Thus, these pawls (32a, 33a) under the stabilizing resilient covers prevent the movement of the mouthpiece cover (2) by interlocking with it on both sides, and therefore inadvertent actuation of the drive mechanism is prevented.

The extensions (32b, 32c; 33b, 33c) under the stabilizing resilient covers pass through the apertures (23c, 23d; 24c, 24d) on the upper and the lower housing members illustrated in figures 5a and 5b and keep the stabilizing resilient covers stable. Namely, the extensions (33b; 33c) under the stabilizing resilient cover that is on the first connection point (29) of the mouthpiece cover pass through the apertures (23c; 23d) on the upper housing member and provide the stabilizing resilient cover (33) to be stably joined with the device. The extensions (32b, 32c) under the stabilizing resilient cover on the second connection point (30) of the mouthpiece cover pass through the apertures (24c, 24d) on the lower housing member and provide the stabilizing resilient cover (32) to be stably joined with the device as illustrated in figure 6f.

Before inhalation, the resilient parts (32d, 33d) of each stabilizing resilient cover illustrated in figures 6c and 6d are pressed on to raise the pawls (32a, 33a) and release the mouthpiece cover (2) in order to actuate the gear mechanism of the device to prepare one dose of dry powder medicament before inhalation. The gear mechanism of the device is actuated and one blister pocket (15a) is opened for one dose of the dry powder medicament to be ready for inhalation when the resilient parts (32d, 33d) of the stabilizing resilient covers are pressed on and the mouthpiece cover (2) is switched from the first position to the second position simultaneously. The necessity to press on the resilient parts (32d, 33d) of the stabilizing resilient covers so as to actuate the gear mechanism preclude the consequences which may result from accidental and inadvertent actuations of the gear mechanism.

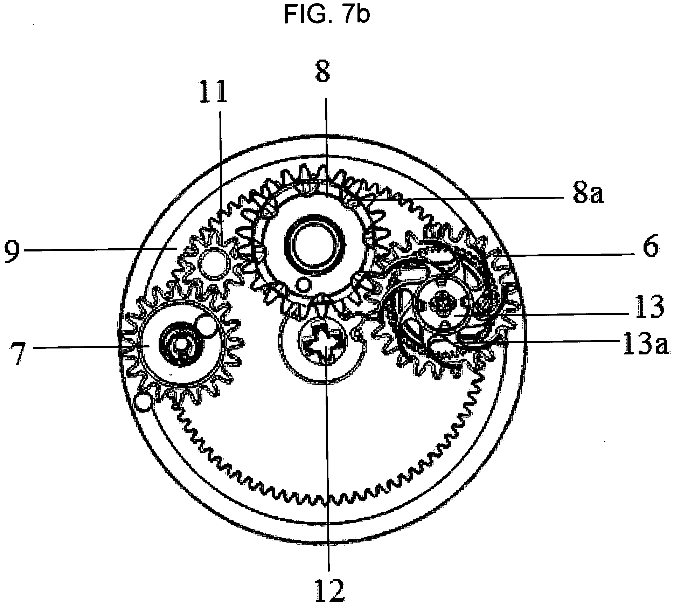

As can be seen in figure 7b, the indexing wheel (8) which synchronizes with the indexing ratchet wheel (3) is engaged with the winding wheel gear (6) and the pinion gear (11) and the rotation of the indexing wheel (8) causes the pinion gear (11) and the winding wheel gear (6) to rotate. Thus, both the peeled lid sheet (15b) of the blister package (15), which is indexed by the rotation of the indexing wheel (8), is tightly coiled on the winding wheel (13) connecting with the winding wheel gear (6) via mechanism wheel (5) and also the counter gear (9) is moved by the pinion gear (11) and the base gear (7) as a result of the rotation of the indexing wheel (8).

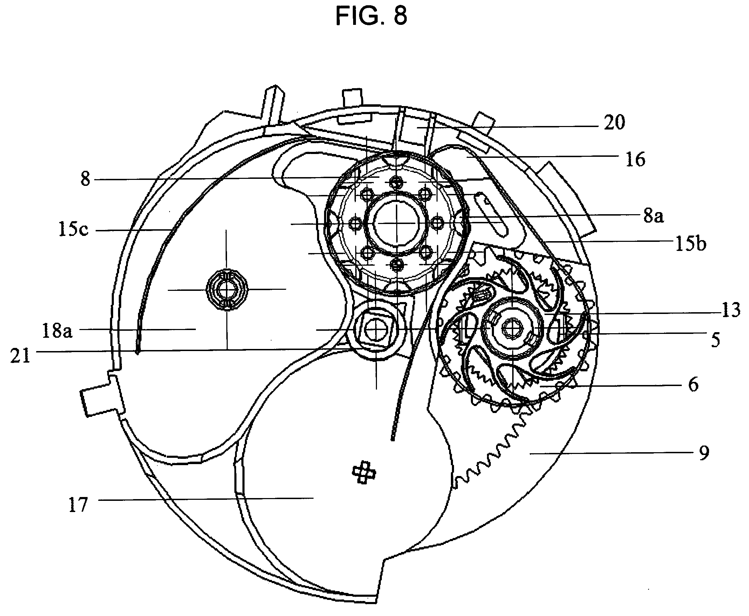

As is seen in figure 8, the lid sheet (15b) of the blister package (15) which is peeled away by the beak (16) and the base sheet (15c) are enclosed in separate compartments of the housing (10). The lid sheet (15b) that provides impermeability of the blister package is indexed over the beak (16) and tightly coiled on the wings (13a) of the winding wheel. The base sheet (15c) of the blister package (15) where the blister pockets (15a), each of which carries one dose of the dry powder medicament, are spaced is accumulated in the separated compartment (18a) of the housing (10). In response to each actuation of the device (1), one dose of the dry powder medicament which is prepared for inhalation after one blister pocket (15a) is opened and air entering the device through the air inlet (22) upon the inhalation of the patient provides to deliver one dose of the dry powder medicament to the patient by entraining it from the blister pocket (15a) to the mouthpiece (14).

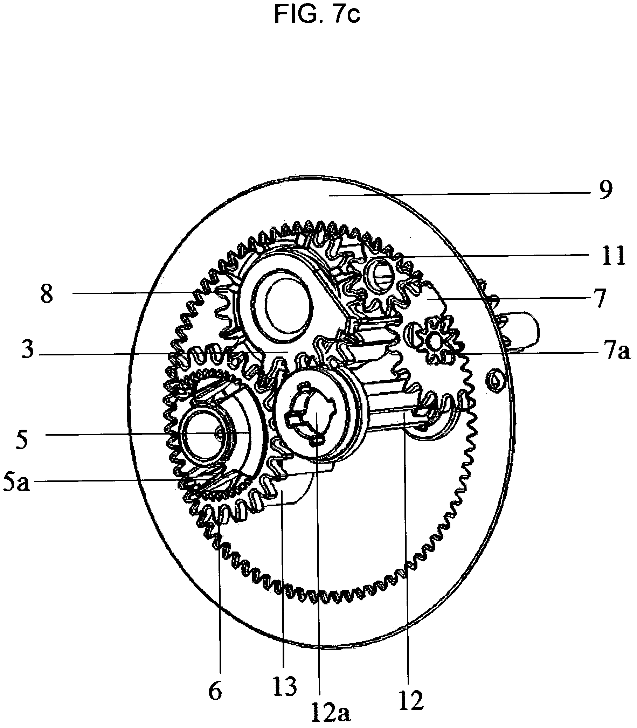

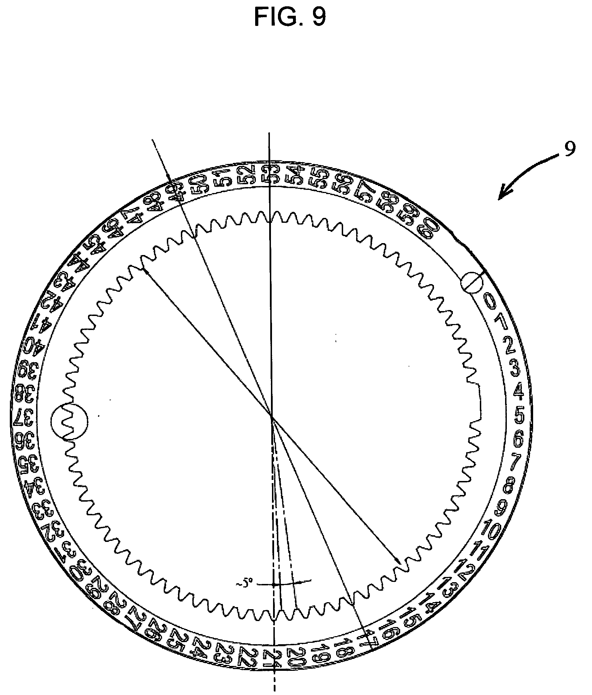

The rotation of the indexing wheel (8) is transmitted by the pinion gear (11) to the base gear (7) engaging with the pinion gear (11) as illustrated in figures 7b and 7c. A small gear which is under a base gear (7) and attached to it engages with the counter gear (9). Thus, the movement of the indexing wheel (8) is transmitted to the counter gear (9) shown in figure 9 by the pinion gear (11) and the base gear. There are numerals incrementing from 1 to 60 in the counter gear (9) displayed in figure 13. The angles between these numerals are all equal and approximately 5°. In response to each actuation of the device, the counter gear rotates approximately 5° and the number of the unused blister pockets remained in the device are clearly seen through the display aperture (4c) on the lower housing member (4b).

In use, of the device described in figures 1-9, the mouthpiece (14) is exposed when the mouthpiece cover (2) is slid from the first position to the second position on the rotational path restricted on both ends by the protrusion parts (23a, 23b, 24a, 24b) on the upper housing member (4a) and the lower housing member (4b); the gear mechanism is triggered by the rotational movement of the mouthpiece cover (2) via the drive gear (12) and one dose of dry powder medicament is prepared for inhalation; the counter gear (9) is indexed and the numeral seen through the display aperture (4c) on the lower housing member (4b) is incremented. After inhalation, the mouthpiece cover (2) is moved from the second position to the first position wherein the mouthpiece (14) is completely covered.

The medicament in dry powder form which is stored in blister cavities is manufactured according to the prior art. According to the present invention, the particle sizes of the active agents comprised in the dry powder medicament are smaller than 20 0m, preferably smaller than 10 µm.

The inhaler utilized in the present invention has been designed so as to deliver the dry powder medicament used in monotherapy or combined therapy. The term "monotherapy" refers to inhalation treatments in which dry powder medicaments comprising a single active agent are used whereas the term "combined therapy" refers to inhalation treatments in which dry powder medicaments comprising more than one active agents are use used.

The dry powder medicament delivered via the device of the present invention comprises at least one excipient in addition to the active agent or agents. These excipients are generally chosen from a group comprising monosaccharides (glucose, arabinose, etc.), disaccharides (lactose, saccharose, maltose, etc.), oligo- and polysaccharides (dextran, etc.), polyalcohols (sorbite, mannite, xylite), salts (sodium chloride, calcium carbonate, etc.) or combinations thereof. According to the present invention, the medicament in dry powder form comprises lactose as the excipient. The medicament in dry powder form comprises fine or coarse excipients particles preferably having various particle size ranges in order to deliver the required amount to the lungs.

The active agent or the active agents comprised in the dry powder medicament which is stored in blister packages used in the device pertaining to the present invention can be selected from a group comprising cromolyns, anti-infectives, antihistamines, anti-inflammatories, bronchodilators, leukotirene inhibitors, PED IV inhibitors, antitussives, diuretics, anticholinergics, hormones, xanthines and pharmaceutically acceptable combinations thereof.

The active agent comprised in the medicament in dry powder form delivered via the inhaler pertaining to the present invention is preferably selected from a group comprising tiotropium, oxitropium, flutropium, ipratropium, glicopironium, flunisolid, beclomethasone, budesonide, fluticasone, mometasone, ciclesonide, rofleponide, dexamethasone, montelukast, methylcyclopropane acetic acid, sodium cromoglicat, nedocromil sodium, Npropylene, teophylline, roflumilast, ariflo (cilomilast), salmeterol, salbutamol, formoterol, terbutaline, carmoterol, indacaterol, cetirizine, levocetirizine, efletirizine, fexofenadine and their racemates, free base, enantiomers or diastereomers and their pharmaceutically acceptable salts, solvates and/or hydrates or a combination of said active agents.

The device pertaining to the present invention is used in administration of the medicament in dry powder form which is utilized in the treatment of many respiratory diseases, particularly in asthma, chronic obstructive pulmonary disorder (COPD) and allergic rhinitis. Accordingly, the respiratory diseases include, but not restricted to, allergic or non-allergic asthma at any phases, acute lung injury (ALI), acute respiratory distress syndrome (ARDS), exacerbation of airways hyperactivity, bronchiectasis, chronic obstructive pulmonary including emphysema and chronic bronchitis, airways or lung diseases (COPD, COAD or COLD), pneumoconiosis, aluminosis, anthracosis, asbestosis, chalicosis, ptilosis, siderosis, silicosis, tabacosis and byssinosis. The device pertaining to the invention can be used in prophylactic or symptomatic treatment. In addition, the medicament in dry powder form which is preferably used in the symptomatic treatment of allergic asthma and COPD is administered to the patient via the device pertaining to the present invention. to deliver one dose of the dry powder medicament to the patient by entraining it from the blister pocket (15a) to the mouthpiece (14).

The rotation of the indexing wheel (8) is transmitted by the pinion gear (11) to the base gear (7) engaging with the pinion gear (11) as illustrated in figures 7b and 7c. A small gear which is under a base gear (7) and attached to it engages with the counter gear (9). Thus, the movement of the indexing wheel (8) is transmitted to the counter gear (9) shown in figure 9 by the pinion gear (11) and the base gear. There are numerals incrementing from 1 to 60 in the counter gear (9) displayed in figure 13. The angles between these numerals are all equal and approximately 5°. In response to each actuation of the device, the counter gear rotates approximately 5° and the number of the unused blister pockets remained in the device are clearly seen through the display aperture (4c) on the lower housing member (4b).

In use, of the device described in figures 1-9, the mouthpiece (14) is exposed when the mouthpiece cover (2) is slid from the first position to the second position on the rotational path restricted on both ends by the protrusion parts (23a, 23b, 24a, 24b) on the upper housing member (4a) and the lower housing member (4b); the gear mechanism is triggered by the rotational movement of the mouthpiece cover (2) via the drive gear (12) and one dose of dry powder medicament is prepared for inhalation; the counter gear (9) is indexed and the numeral seen through the display aperture (4c) on the lower housing member (4b) is incremented. After inhalation, the mouthpiece cover (2) is moved from the second position to the first position wherein the mouthpiece (14) is completely covered.

The medicament in dry powder form which is stored in blister cavities is manufactured according to the prior art. According to the present invention, the particle sizes of the active agents comprised in the dry powder medicament are smaller than 20 µm, preferably smaller than 10 µm.

The inhaler utilized in the present invention has been designed so as to deliver the dry powder medicament used in monotherapy or combined therapy. The term "monotherapy" refers to inhalation treatments in which dry powder medicaments comprising a single active agent are used whereas the term "combined therapy" refers to inhalation treatments in which dry powder medicaments comprising more than one active agents are use used.

The dry powder medicament delivered via the device of the present invention comprises at least one excipient in addition to the active agent or agents. These excipients are generally chosen from a group comprising monosaccharides (glucose, arabinose, etc.), disaccharides (lactose, saccharose, maltose, etc.), oligo- and polysaccharides (dextran, etc.), polyalcohols (sorbite, mannite, xylite), salts (sodium chloride, calcium carbonate, etc.) or combinations thereof. According to the present invention, the medicament in dry powder form comprises lactose as the excipient. The medicament in dry powder form comprises fine or coarse excipients particles preferably having various particle size ranges in order to deliver the required amount to the lungs.

The active agent or the active agents comprised in the dry powder medicament which is stored in blister packages used in the device pertaining to the present invention can be selected from a group comprising cromolyns, anti-infectives, antihistamines, anti-inflammatories, bronchodilators, leukotirene inhibitors, PED IV inhibitors, antitussives, diuretics, anticholinergics, hormones, xanthines and pharmaceutically acceptable combinations thereof.

The active agent comprised in the medicament in dry powder form delivered via the inhaler pertaining to the present invention is preferably selected from a group comprising tiotropium, oxitropium, flutropium, ipratropium, glicopironium, flunisolid, beclomethasone, budesonide, fluticasone, mometasone, ciclesonide, rofleponide, dexamethasone, montelukast, methylcyclopropane acetic acid, sodium cromoglicat, nedocromil sodium, Npropylene, teophylline, roflumilast, ariflo (cilomilast), salmeterol, salbutamol, formoterol, terbutaline, carmoterol, indacaterol, cetirizine, levocetirizine, efletirizine, fexofenadine and their racemates, free base, enantiomers or diastereomers and their pharmaceutically acceptable salts, solvates and/or hydrates or a combination of said active agents.

The device pertaining to the present invention is used in administration of the medicament in dry powder form which is utilized in the treatment of many respiratory diseases, particularly in asthma, chronic obstructive pulmonary disorder (COPD) and allergic rhinitis. Accordingly, the respiratory diseases include, but not restricted to, allergic or non-allergic asthma at any phases, acute lung injury (ALI), acute respiratory distress syndrome (ARDS), exacerbation of airways hyperactivity, bronchiectasis, chronic obstructive pulmonary including emphysema and chronic bronchitis, airways or lung diseases (COPD, COAD or COLD), pneumoconiosis, aluminosis, anthracosis, asbestosis, chalicosis, ptilosis, siderosis, silicosis, tabacosis and byssinosis. The device pertaining to the invention can be used in prophylactic or symptomatic treatment. In addition, the medicament in dry powder form which is preferably used in the symptomatic treatment of allergic asthma and COPD is administered to the patient via the device pertaining to the present invention.

## Claims

1. An inhaler (1) suitable for delivery of the medicament in dry powder form from a blister package (15) composed of a plurality of blister pockets (15a) each of which comprises medicament in dry powder form and which are spaced at equal intervals, the inhaler comprising:
- a mouthpiece (14) enabling the patient to inhale one dose of the medicament in dry powder form from the opened blister pocket in use,
- a gear mechanism enabling, in use, a blister package (15) to be indexed and the medicament in dry powder form to become ready for inhalation,
- a rotatable mouthpiece cover (2) covering the mouthpiece (14) and triggering the gear mechanism,
- a drive gear (12) which is a component of the gear mechanism that is joined with the mouthpiece cover (2) via side covers (31a, 31c), and
- a housing (10) situated between an upper housing member (4a) and a lower housing member (4b) in which a blister package and the gear mechanism are enclosed in use,
the inhaler **characterized by** side covers (31a, 31c) which provide a connection between each end (12a; 12b) of the drive gear and an appropriate connection point (30; 29) of the mouthpiece cover so as to enable the drive gear (12) and the mouthpiece cover (2) to synchronize, and at least one stopper (26) that hinders the rotation of at least one gear in the gear mechanism.

2. The inhaler (1) according to claim 1, wherein the stopper component (26) is positioned in the lower housing member (4b) and prevents the rotation of the gear mechanism by interlocking with the tooth of at least one gear.

3. The inhaler (1) according to claim 1 or 2, wherein the stopper (26) hinders the rotation of the indexing ratchet wheel (3) of the gear mechanism.

4. The inhaler (1) according to claim 1, wherein each end (12a; 12b) of the drive gear is carved such that the ends (31b; 31d) of the side covers can engage therewith.

5. The inhaler (1) according to claim 4, wherein the inner surfaces of the carved parts (12a; 12b) on each end of the drive gear match with the shapes of the ends (31b; 31d) of the side covers attached to each end.

6. The inhaler (1) according to claim 1, wherein each side cover (31a; 31c) passes through the connection point (30; 29) of the mouthpiece cover, attaches the carved part (12a; 12b) on the end of the drive gear and interconnects the mouthpiece cover (2) and the drive gear.

7. The inhaler (1) according to claim 1, wherein a restricted path for the rotational movement of the mouthpiece cover is provided by protrusions (23a, 23b; 24a, 24b) on the upper (4a) and lower (4b) housing members.

8. The inhaler (1) according to claim 1, wherein the mouthpiece cover (2) is rotationally moved by being slid on the upper (4a) and the lower (4b) housing member.

9. The inhaler (1) according to any one of the preceding claims, wherein said mouthpiece cover (2) rotates along a constant-distance path that recesses (23a, 23b; 24a, 24b) of the upper and the lower housing members define by a fixed angle in the range of 30° to 160° according to the shape of the device, in response to the each actuation of the device.

10. The inhaler (1) according to any one of the preceding claims, wherein the mouthpiece cover (2) can be in one of two positions:
one where the mouthpiece (14) is completely covered and the device (1) is on standby mode when the mouthpiece cover (2) is leaning on a protrusion (23a, 24a) in one end of the rotational path,
and the other where one dose of the medicament in dry powder form is ready for inhalation in use upon the actuation of the device (1) when the mouthpiece cover (2) is leaning on a protrusion (23a, 24a) in the other end of the rotational path.

11. The inhaler (1) according to claim 1, wherein all components of the gear mechanism directly or indirectly engage with each other.

12. The inhaler (1) according to either one of claim 1 or 11, wherein said gear mechanism is composed of;
- a drive gear (12) which actuate the device (1) by transmitting the constant-angle movement of the mouthpiece cover (2) to an indexing ratchet wheel (3);
- an indexing wheel (8) which synchronizes with the indexing ratchet wheel (3) and enables the blister package (15) to be indexed in use;
- a winding wheel gear (6) which moves a winding wheel (13) via a mechanism wheel (5) upon rotation of the indexing wheel (8);
- a pinion gear (11) and a base gear (7) that transmit the movement of the indexing wheel (8) to the counter wheel (9);
- a counter gear (9) which displays the number of the unused blister pockets (15a) remained in the device (1).

13. The inhaler (1) according to claim 12, wherein there are 8 recesses on the indexing wheel (8) that is a component of the gear mechanism.

14. The inhaler (1) according to claim 13, wherein the rotation of the indexing wheel (8) by a constant angle of 45° in response to each actuation of the device (1) is provided by the synchronization of the mouthpiece cover (2) that rotates by a constant angle in the range of 30° to 160° depending on the shape of the device, with the drive gear (12) that attaches with the connection points (30; 29) of the mouthpiece cover via the side covers (31a; 31c).

15. An inhaler according to any proceeding claim further comprising a blister package (15) composed of a plurality of blister pockets (15a), each of which comprises medicament in dry powder form and which are spaced at equal intervals.

## Patentansprüche

1. Ein Inhalator (1), der für die Abgabe des Medikaments in Form eines trockenen Pulvers aus einer Blisterverpackung (15) geeignet ist, die aus einer Vielzahl von Blistertaschen (15a) besteht, von denen jede ein Medikament in Form eines trockenen Pulvers umfasst und die in gleichen Abständen beabstandet sind; der Inhalator umfasst die Folgenden;
- ein Mundstück (14), das es den Patienten ermöglicht, das Medikament in trockener Pulverform aus einer geöffneten Blistertasche im Einsatz zu inhalieren,
- ein Getriebe, das es bei Gebrauch einer Blisterverpackung (15) es ermöglicht, indiziert zu werden und, das den Medikamenten in trockener Pulverform es ermöglicht, zur Inhalation bereit zu werden,
- eine drehbare Mundstückabdeckung (2), die das Mundstück (14) abdeckt und das Getriebe auslöst,
- ein Antriebsrad (12), das ein Bestandteil des Getriebes ist, das mit der Mundstückabdeckung (2) durch Seitendeckel (31 a, 31 c) verbunden ist, und
- ein Gehäuse (10) zwischen einem oberen Gehäuseelement (4a) und einem unteren Gehäuseelement (4b), in dem die Blisterverpackung und das Getriebe eingeschlossen sind;
Inhalator, der durch die Seitendeckel (31a, 31c) gekennzeichnet wird und eine Verbindung zwischen jedem Ende (12a; 12b) des Antriebsrades und einen entsprechenden Verbindungspunkt (30; 29) der Mundstückabdeckung bietet, damit das Antriebsrad (12) und die Mundstückabdeckung (2) synchronisiert werden können; und mindestens einen Stopfen (26), der die Drehung von mindestens einem Zahnrad im Getriebe verhindert.

2. Inhalator (1) nach Anspruch 1, wobei der Stopfenteil (26) in dem unteren Gehäuseelement (4b) positioniert ist und die Drehung des Getriebes durch Verriegeln mit dem Zahn von mindestens einem Zahnrad verhindert.

3. Inhalator (1) nach Anspruch 1 oder 2, wobei der Stopfen (26) die Drehung des Indizierungssperrrades (3) des Getriebes verhindert.

4. Inhalator (1) nach Anspruch 1, wobei jedes Ende (12a; 12b) des Antriebsrades geschnitzt wird, damit die Enden (31b, 31d) der Seitendeckel in Eingriff sein können.

5. Inhalator (1) nach Anspruch 4, wobei die Innenflächen der geschnitzten Teile (12a; 12b) an beiden Enden des Antriebsrades mit den Formen der Enden (31b; 31d) der Seitendeckel übereinstimmen, die an jedem Ende befestigt sind.

6. Inhalator (1) nach Anspruch 1, wobei jeder Seitendeckel (31a; 31c) durch den Verbindungspunkt (30; 29) der Mundstückabdeckung läuft, den geschnitzten Teil (12a; 12b) am Ende des Antriebsrades befestigt und die Mundstückabdeckung (2) und das Antriebsrad verbindet.

7. Inhalator (1) nach Anspruch 1, wobei ein eingeschränkter Pfad für die Drehbewegung der Mundstückabdeckung durch Vorsprünge (23a, 23b; 24a, 24b) auf den oberen (4a) und den unteren (4b) Gehäuseelementen vorgesehen wird.

8. Der Inhalator (1) nach Anspruch 1, wobei die Mundstückabdeckung (2) sich dreht, indem sie auf den oberen (4a) und den unteren (4b) Gehäuseelementen geschoben wird.

9. Der Inhalator (1) nach einem der vorhergehenden Ansprüche, wobei die genannte Mundstückabdeckung (2) sich entlang eines konstanten Abstandes dreht, so dass Aussparungen (23a, 23b; 24a, 24b) der oberen und unteren Gehäuseelemente um einen festen Winkel im Bereich von 30° bis 160° je nach der Form des Gerätes in Reaktion auf die jede Betätigung des Geräts definiert werden.

10. Der Inhalator (1) nach einem der vorhergehenden Ansprüche, wobei die Mundstückabdeckung (2) nur in zwei Positionen liegen kann:
bei der erster Position ist das Mundstück (14) vollständig abgedeckt und das Gerät (1) ist im Standby-Modus, wenn die Mundstückabdeckung (2) an einen Vorsprung (23 a, 24a) an einem Ende des Drehpfades anlehnt;
und bei der zweiten Position wird eine Dosis des Trockenpulvermedikaments als Reaktion auf die Betätigung des Geräts (1) zur Inhalation bereit, wenn die Mundstückabdeckung (2) an einen Vorsprung (23a, 24a) am anderen Ende des Drehpfades anlehnt.

11. Der Inhalator (1) nach Anspruch 1, wobei alle Komponente des Getriebes direkt oder indirekt miteinander in Eingriff stehen.

12. Der Inhalator (1) nach Anspruch 1 oder 11, wobei der Antriebsmechanismus die Folgenden umfasst;
- ein Antriebsrad (12), das das Gerät (1) durch Übertragen der konstanten Winkelbewegung der Mundstückabdeckung (2) an ein Indizierungssperrrad (3) auslöst;
- ein Indizierungsrad (8), das mit dem Indizierungssperrrad (3) synchronisiert wird und der Blisterverpackung (15) es ermöglicht, im Gebrauch indiziert zu werden;
- ein Wickelzahnrad (6), das das Wickelrad (13) über den Radmechanismus (5) beim Drehen des Indizierungsrades (8) bewegt;
- ein Ritzel (11) und Basiszahnrad (7), die die Bewegung des Indizierungsrades (8) zu dem Gegenzahnrad (9) übertragen;
- ein Gegenzahnrad (9), das die Anzahl der nicht verwendeten und im Gerät (1) verbleibender Blistertaschen (15a) zeigt.

13. Inhalator (1) nach Anspruch 12, wobei es 8 Ausnehmungen auf dem Indizierungsrad (8) gibt, das eine Komponente des Getriebes ist.

14. Inhalator (1) nach Anspruch 13, wobei die Drehung des Indizierungsrades (8) um einen konstanten Winkel von 45° als Reaktion auf jede Betätigung des Geräts (1) durch die Synchronisierung der Mundstückabdeckung (2) vorgesehen wird, die sich um einen konstanten Winkel im Bereich von 30° bis 160° in Abhängigkeit von der Form des Gerätes mit dem Antriebsrad (12) dreht, das mit den Verbindungspunkten (30; 29) der Mundstückabdeckung durch die Seitendeckel (31 a; 31 c) befestigt wird.

15. Inhalator nach einem der vorstehenden Ansprüche ferner umfassend eine Blisterpackung (15), die aus einer Vielzahl von Blistertaschen (15a) besteht, von denen jede ein Medikament in Form eines trockenen Pulvers umfasst und die in gleichen Abständen beabstandet sind.

## Revendications

1. Un inhalateur (1) approprié pour la livraison du médicament sous la forme de poudre sèche à partir d'un emballage coque (15) composée d'une pluralité de poches blisters (15a) dont chacune comprend le médicament sous la forme de poudre sèche et qui sont espacées à des intervalles égaux ; ledit inhalateur comprenant :
- un embout (14) permettant au patient d'inhaler une dose du médicament sous la forme de poudre sèche depuis la poche blister ouvert en cours d'utilisation,
- un mécanisme d'engrenage permettant en cours d'utilisation un emballage blister (15) à être indexé et le médicament sous la forme de poudre sèche à être prêt pour l'inhalation,
- une couverture rotative de l'embout (2) couvrant l'embout (14) et déclenchant le mécanisme d'engrenage,
- un engrenage d'entrainement (12) qui est un composant du mécanisme d'engrenage qui est relié à la couverture de l'embout (2) via les couvertures latérales (31a, 31c), et
- un boîtier (10) situé entre l'élément de boîtier supérieur (4a) et l'élément de boîtier inférieur (4b), dans lequel un emballage blister et le mécanisme d'engrenage sont enfermés en cours d'utilisation,
l'inhalateur est **caractérisé en ce que**,
les couvertures latéraux (31a, 31c) qui assurent une connexion entre chacune des extrémités (12a; 12b) de l'engrenage d'entraînement et un point de connexion approprié (30; 29) de la couverture de l'embout afin de permettre l'engrenage d'entraînement (12) et la couverture de l'embout (2) à être synchroniser,
et au moins une butée (26) qui empêche la rotation d'au moins un engrenage dans le mécanisme d'engrenage.

2. L'inhalateur (1) selon la revendication 1, dans lequel le composant de butée (26) est positionné dans l'élément de boîtier inférieur (4b) et empêche la rotation de l'engrenage par emboîtement avec la dent d'au moins une roue dentée.

3. L'inhalateur (1) selon les revendications 1 ou 2, dans lequel la butée (26) empêche la rotation de la roue programmatrice d'indexation (3) dans le mécanisme d'engrenage.

4. L'inhalateur (1) selon la revendication 1, dans lequel chaque extrémité (12a; 12b) de l'engrenage d'entraînement est gravé de telle sorte que les extrémités (31b; 31d) des couvertures latéraux peuvent s'engager avec celui-ci.

5. L'inhalateur (1) selon la revendication 4, dans lequel les surfaces intérieures des parties gravées (12a, 12b) sur chaque extrémité de l'engrenage d'entraînement concordent avec les formes des extrémités (31b; 31d) des couvertures latéraux fixées à chaque extrémité.

6. L'inhalateur (1) selon la revendication 1, dans lequel chaque couverture latérale (31a; 31c) traverse le point de connexion (30; 29) de la couverture de l'embout, attache la partie gravée (12a; 12b) sur l'extrémité de l'engrenage d'entraînement et interconnecte la couverture de l'embout (2) et l'engrenage d'entraînement.

7. L'inhalateur (1) selon la revendication 1, dans lequel une route restreinte pour le mouvement rotative de la couverture de l'embout est prévue par les saillies (23a, 23b; 24a, 24b) sur les éléments de boîtier supérieur (4a) et inférieur (4b).

8. L'inhalateur (1) selon la revendication 1, dans lequel la couverture de l'embout (2) est déplacée par rotation par glissement sur l'élément de boîtier supérieur (4a) et inférieur (4b).

9. L'inhalateur (1) selon l'une quelconque des revendications précédentes, dans lequel ladite couverture de l'embout (2) tourne le long d'une route à distance constante que les cavités (23a, 23b; 24a, 24b) de l'élément de boîtier supérieur et inférieur définie par un angle fixe dans la plage de 30 ° à 160 °, suivant la forme du dispositif, en réponse à chaque actionnement du dispositif.

10. L'inhalateur (1) selon quelconque des revendications précédentes dans lequel la couverture de l'embout (2) peut être dans une position sur deux :
celle où l'embout (14) est couvert complètement et l'appareil (1) est en mode veille quand la couverture de l'embout (2) se penche sur une saillie (23a, 24a) en une extrémité de la route de rotation,
et celle où une dose de médicament sous la forme de poudre sèche est prête pour l'inhalation en utilisation sur la mise en marche de l'appareil (1) quand la couverture de l'embout (2) se penche sur une saillie (23a, 24a) en l'autre extrémité de la route de rotation.

11. L'inhalateur (1) selon la revendication 1, dans lequel tous les composants du mécanisme d'engrenage s'engagent les uns avec les autres directement ou indirectement.

12. L'inhalateur (1) selon l'une quelconque des revendications 1 ou 11, dans lequel ledit mécanisme d'engrenage est composé de :
- un engrenage d'entrainement (12) qui enclenche l'appareil (1) en transmettant le mouvement d'un angle constant de la couverture de l'embout (2) à une roue à rochet d'indexation (3) ;
- une roue d'indexation (8) qui synchronise avec la roue à rochet d'indexation (3) et permet l'emballage coque (15) à être indexé en cours d'utilisation ;
- un engrenage de roue d'enroulement (6) qui déplace une roue d'enroulement (13) par un mécanisme de roue (5) sur la rotation de la roue d'indexation (8) ;
- un engrenage à pignons (11) et un engrenage de base (7) qui transmettent le mouvement du la roue d'indexation (8) à la roue de contrepoussée (9) ;
- une roue de contrepoussée (9) qui affiche le nombre des poches blisters (15a) inutilisées restantes dans l'appareil (1).

13. L'inhalateur (1) selon la revendication 12, dans lequel il y a 8 cavités sur la roue d'indexation (8) qui est un composant du mécanisme d'engrenage.

14. L'inhalateur (1) selon la revendication 13, dans lequel la rotation de la roue d'indexation (8) par un angle constant de 45° en réponse à chaque actionnement du dispositif (1) est prévu par le synchronisation de la couverture de l'embout (2) qui tourne par un angle constant dans la plage de 30° à 160° en fonction de la forme du dispositif, avec l'engrenage d'entrainement (12) qui s'attache avec les points de connexion (30; 29) de la couverture de l'embout via les couvertures latérales (31a; 31c).

15. Un inhalateur selon l'une quelconque des revendications précédentes comprenant en outre un emballage blister (15) qui est composé d'une pluralité des poches blisters (15a), chacune desquelles comprenant le médicament sous la forme de poudre sèche et qui sont espacées à des intervalles égaux.
